# FASCICULE DE BREVET EUROPEEN

(11) **EP 2 211 834 B1**
(45) Date de publication et mention de la délivrance du brevet: **11.02.2026**
(21) Numéro de dépôt: 08842571.5
(22) Date de dépôt: 06.10.2008
(51) Int. Cl.: A61K 9/127, A61K 47/32, A61K 47/44, A61K 9/00, A61P 31/04, A61P 31/12, A61P 33/00, A61P 37/04

(54) **PROCEDE DE PREPARATION D'UNE COMPOSITION VACCINALE COMPRENANT AU MOINS UN ANTIGENE ET AU MOINS UN ADJUVANT**
VERFAHREN ZUR HERSTELLUNG EINER IMPFSTOFFZUSAMMENSETZUNG MIT MINDESTENS EINEM ANTIGEN UND MINDESTENS EINEM ADJUVANS
METHOD FOR PREPARING A VACCINE COMPOSITION COMPRISING AT LEAST ONE ANTIGEN AND AT LEAST ONE ADJUVANT

(30) Priorité: 24.10.2007 FR 0758542
(43) Date de publication de la demande: 04.08.2010
(73) Titulaire: SOCIETE D'EXPLOITATION DE PRODUITS POUR LES INDUSTRIES CHIMIQUES SEPPIC, 75321 Paris Cedex 07 (FR)
(72) Inventeur: DUPUIS, Laurent, F-51100 Reims (FR); GAUCHERON, Jérôme, F-81100 Castres (FR); BRAUN, Olivier, F-81100 Castres (FR)
(74) Mandataire: Air Liquide
(86) Numéro de dépôt international: PCT/FR2008/051807
(87) Numéro de publication internationale: WO 2009/053601

(56) Documents cités:
- WO-A2-03/024354
- WO-A2-98/17310
- US-A1- 2001 028 884
- US-A1- 2001 028 884
- US-A1- 2004 170 640
- US-A1- 2004 170 640

## Description

La présente invention concerne un procédé de préparation d'une composition vaccinale comprenant au moins un antigène, notamment un antigène d'origine virale, bactérienne ou parasitaire et au moins un adjuvant selon la revendication 1.

Le développement de vaccins inactivés ou contenant des antigènes purifiés est de plus en plus important, car il permet d'éviter les effets secondaires indésirables chez le sujet traité. Cependant, l'amélioration de la qualité des antigènes se fait au détriment de leur caractère immunogène. C'est pour cette raison qu'ils sont associés à des adjuvants d'immunité.

Les adjuvants d'immunité sont des produits qui augmentent les réactions du système immunitaire, lorsqu'ils sont administrés en présence d'antigènes d'origine virale, bactérienne ou synthétique. Ils provoquent l'apparition massive de macrophages au site d'injection, puis dans les nodules lymphatiques, accroissent la production d'immunoglobulines spécifiques, les anticorps, et stimulent de nombreuses cellules impliquées dans les mécanismes de la défense immunitaire.

Ces adjuvants sont de natures diverses. Ils peuvent par exemple consister en des émulsions se présentant sous la forme d'émulsions eau-dans-huile E/H, ou huile-dans-eau H/E, ou eau-dans-huile-dans-eau E/H/E, ou huile-dans-eau-dans-huile H/E/H.

Les adjuvants de Freund sont très efficaces ; ils résultent de l'association d'une huile minérale et d'un ester de mannitol contenant ou non une mycobactérie tuée. Les vaccins réalisés par mélange en parts égales d'un adjuvant de Freund avec un milieu antigénique aqueux, restent encore les références dans le monde entier pour les études en laboratoire. Ils se présentent sous forme d'émulsions eau dans huile E/H, c'est à dire d'émulsions pour lesquelles la phase continue est constituée par une huile ou un mélange d'huiles, et la phase dispersée est une phase aqueuse pouvant comprendre des excipients solubilisants comme par exemple le glycérol ou le diméthylsulfoxyde. Le principe actif est généralement localisé dans la phase aqueuse qui est souvent une solution saline tamponnée. Cette phase est sous forme de gouttes séparées entre elles par un film huileux. Cette formulation permet d'obtenir des réponses biologiques amplifiées et prolongées dans le temps.

Cependant les émulsions de ce type E/H sont généralement très visqueuses et sont donc difficilement injectables. Elles nécessitent souvent l'utilisation de seringues à diamètre d'aiguille important et sont la cause de douleurs lors de l'injection et de traumatismes au niveau du site de l'injection.

La teneur massique en phase aqueuse des émulsions E/H injectables se situe autour de 30 % à 40 % pour 100% de la masse de l'émulsion. La teneur massique maximale rencontrée est 50 % massique. Or cette limite constitue un frein notamment au développement de vaccins polyvalents, dans lesquels sont associés plusieurs antigènes et pour lesquels il serait préférable d'obtenir des émulsions E/H ayant des teneurs en phase aqueuse supérieures à 50 % massique.

Or, dans une émulsion E/H à faible teneur en phase aqueuse, jusqu' à environ 20 % massique, la viscosité de l'émulsion est très proche de la viscosité de l'huile. L'augmentation de la proportion massique en phase aqueuse augmente sa viscosité. C'est ainsi qu'une émulsion à 20 % massique d'eau ayant une viscosité de 100 mPa.s, mesurée à l'aide d'un viscosimètre BROOKFIELD LVT équipé d'un mobile N°2 tournant à une vitesse de 60 tours par minute, se transforme en une crème très difficilement injectable lorsqu'on augmente la teneur en eau jusqu'à 50 % massique.

Certains adjuvants huileux commerciaux se présentant sous la forme d'émulsions E/H, comme le MONTANIDE^{™} ISA 70 permettent d'obtenir des émulsions injectables E/H contenant environ 30 % en poids, de phase aqueuse, 70 % en poids de phase huileuse et ayant des viscosités de l'ordre de 50 à 100 mPa.s, mesurée à l'aide d'un viscosimètre BROOKFIELD LVT équipé d'un mobile N°2 tournant à une vitesse de 60 tours par minute,. D'autres adjuvants, comme le MONTANIDE^{™} ISA 50V2, permettent d'obtenir des émulsions injectables E/H contenant environ 50 % en poids de phase aqueuse, 50 % en poids de phase huileuse et ayant des viscosités inférieures à 250 mPa.s mesurée à l'aide d'un viscosimètre BROOKFIELD LVT équipé d'un mobile N°2 tournant à une vitesse de 60 tours par minute,.

La demande internationale de brevet publiée sous le numéro WO 99/20305 divulgue l'utilisation de tensioactifs sur oléate de mannitol avec des huiles minérales comme le MARCOL^{™} 52 pour faire des émulsions, dites fluides, de viscosités de l'ordre de 500 mPa.s mesurée à l'aide d'un viscosimètre BROOKFIELD LVT équipé d'un mobile N°2 tournant à une vitesse de 60 tours par minute.

La demande internationale de brevet publiée sous le numéro WO 98/17310 décrit des compositions adjuvantes à base de polymères polyanioniques solubles dans l'eau ayant des unités de répétition constitutionnelles anioniques qui peuvent être identiques ou différentes, ou des polymères polyanioniques ayant des unités de répétition constitutionnelles anioniques (identiques ou différentes) et des unités de répétition constitutionnelles hydrophobes.

La demande de brevet publiée sous le numéro US 2004/170640 A1 décrit un procédé de préparation d'une composition d'adjuvant comprenant un mélange de lécithine et d'un polymère ou copolymère acrylique.

La demande de brevet publiée sous le numéro WO 03/024354 décrit des compositions d'adjuvants comprenant des copolymères d'acide acrylique, plus particulièrement une émulsion de latex d'un copolymère acrylique aqueux non coalescé d'acide acrylique, d'acide méthacrylique mélangé avec du styrène. La demande de brevet publiée sous le numéro US 2001/028884 A1 décrit l'utilisation d'un polymère d'acide acrylique ou d'acide méthacrylique, d'un polymère d'anhydride maléique et d'un dérivé alcényle pour adjuver des compositions immunogènes ou des vaccins, qui peuvent être formulés sous la forme d'une émulsion huile dans l'eau.

La notion d'émulsion fluide dépend largement du domaine d'application. Pour les émulsions injectables, la limite supérieure de viscosité pour une émulsion fluide est définie par rapport à la viscosité d'une émulsion de type E/H de référence, qui contient 50 % massique de phase aqueuse et 50 % massique d'adjuvant incomplet de Freund (IFA) ; cette viscosité, mesurée à l'aide d'un viscosimètre BROOKFIELD LVT équipé d'un mobile N°3 tournant à une vitesse de 30 tours par minute, est de l'ordre d'environ 2 000 mPa.s. Cette émulsion est considérée comme très visqueuse.

Une émulsion de type E/H sera dite fluide, si sa viscosité est inférieure à 4 fois celle de cette émulsion de référence, c'est-à-dire inférieure à 500 mPa.s à 25°C, mesurée à l'aide d'un viscosimètre BROOKFIELD LVT équipé d'un mobile N°2 et tournant à une vitesse de 30 tours par minute.

Cependant les émulsions fluides sont en général moins stables que les émulsions plus visqueuses, puisque des déphasages à température ambiante sont observés quelques jours seulement, après leur préparation.

Il existe des polymères épaississants de phases aqueuses se présentant sous forme de poudres, tels que les homopolymères de l'acide acrylique sous leurs formes sodiques ou les copolymères à base d'acide acrylique et de ses esters. On citera par exemple, les polymères commercialisés par la société Noveon sous le nom de marque, CARBOPOL^{™} et PEMULEN^{™}. Ils sont décrits notamment dans les brevets américains US 5,373,044, US 2,798,053 et dans le brevet européen EP 0 301 532. Ces polymères ont été développés à l'origine comme épaississants destinés à épaissir des formulations essentiellement destinées à des applications cosmétiques, et qui sont décrits et utilisés depuis de nombreuses années. Ces polymères sont obtenus à partir d'un monomère tel que par exemple l'acide acrylique, l'acide méthacrylique, les esters de l'acide acrylique ou de l'acide méthacrylique en solution dans une phase solvant organique. Au cours de la réaction de polymérisation, obtenue par l'ajout de différents catalyseurs dans des conditions de température et de pression spécifiques, le polymère s'insolubilise de la phase solvant initiale pour précipiter au fond du réacteur. Ce procédé est appelé « polymérisation par précipitation ».

Les polymères ainsi obtenus, dont les grades les plus connus sont les CARBOPOL^{®}, sont des épaississants très efficaces très utilisés en cosmétique. Ces polymères sont aussi utilisés pour des applications pharmaceutiques variées :
- Préparation de matrices de polymères pour effet de libération retardée,
- Préparation de gels de polymères, et complexation desdits polymères avec des protéines,
- Préparation de films de polymères induisant une protection lors de la mise en contact avec les fluides biologiques,
- Préparation de formulations bio-adhésives assurant une plus grande rémanence lors de l'application sur les muqueuses,
- Préparation d'adjuvants de vaccins.

L'utilisation comme adjuvant de vaccin, avec des teneurs massiques finales en polymère de l'ordre du « pour cent », se présente sous la forme d'un vaccin fluide et translucide facilement injectable.

Les propriétés adjuvantes des substances synthétiques sont très liées à leurs formes physiques lors de l'administration. Ainsi, la taille des particules utilisées pour déclencher un effet adjuvant important est un paramètre majeur qui va contrôler la possibilité de prise en charge par les cellules immunocompétentes. De plus, en fonction de la taille des particules, il sera aussi possible d'orienter la réponse vers une production d'anticorps (réponse humorale) ou plutôt pour stimuler des cellules spécifiques du système immunitaire (réponse cellulaire). La synthèse de microsphères de différents polymères en phase solvant sous forme de dispersions est largement décrite dans la littérature avec possibilité d'encapsulation d'un agent biologiquement actif (pouvant être un antigène dans le cas des vaccins) ou utilisé comme support d'adsorption pour véhiculer et présenter un agent actif lié à la surface extérieure des microsphères par des interactions plus ou moins fortes.

La maîtrise de la taille des particules adjuvantes est un défi permanent car elle doit être accompagnée d'une stabilité de ces particules adjuvantes au cours du temps. Il est important notamment d'éviter les agrégations au cours du stockage ou les séparations de phases. La maîtrise de la taille doit également être accompagnée d'une stabilité de la composition, après injection dans l'être vivant, notamment en raison de stress liés à la variation de pH, à la présence d'enzymes, aux variations de température.

C'est pourquoi, la demanderesse a cherché à mettre au point une technologie originale de synthèse des polymères permettant de contrôler la taille des agglomérats de polymères (ou «microgels») assurant ainsi de très bonnes performances adjuvantes. Pour cela, la technologie de la polymérisation en émulsion eau dans huile E/H, ou en émulsion inverse, est utilisée pour préparer ces polymères - comme divulgué dans la revendication 1. En effet, la taille des gouttes de solution de monomère est contrôlée par l'utilisation de systèmes tensioactifs émulsionnants de type eau-dans-huile adaptés aux technologies des émulsions E/H. Il s'agit en général du couplage d'un tensioactif émulsionnant lipophile, ayant par exemple un HLB compris entre 2 et 7, avec un tensioactif émulsionnant hydrophile, ayant par exemple un HLB compris entre 10 et 15, avec une valeur moyenne pondérée assurant une émulsion E/H homogène. La polymérisation des monomères est assurée par l'ajout des catalyseurs et autres réticulants aux concentrations habituelles dans des conditions de pH et de température adaptées à une réaction complète du monomère pour former des polymères.

La dimension des particules adjuvantes a un impact direct sur les propriétés immunologiques des formulations les contenant. De plus, en fonction des espèces devant être vaccinées et du type de vaccin considéré, la taille optimale des particules peut différer. Par le savoir-faire de formulation des émulsions (optimisation du HLB, nature des tensioactifs, concentration, procédé) il est possible lors du procédé de synthèse en émulsion inverse de maîtriser la répartition de la taille des gouttes de l'émulsion et ainsi la taille moyenne et la répartition granulométrique des agglomérats. Par dispersion, on entend, en fonction de la teneur en tensioactif et de l'énergie de cisaillement fournie lors de l'étape d'émulsification, des systèmes hétérogènes pouvant être :
- des suspensions,
- des émulsions,
- des microémulsions.

Ces différentes formes dispersées permettent de maîtriser la dispersion de taille des microgels dans des domaines allant de 50 nm à 100 µm.

Les conditions chimiques de synthèse du polymère (types de monomères, catalyseurs, initiateurs) permettent de contrôler les propriétés individuelles du polymère obtenu (masse molaire moyenne, réticulation, caractère anionique).

L'adjuvant ainsi synthétisé se présente sous la forme d'une émulsion « gouttes de polymère dans huile » ou « latex inverse ». Cette émulsion, lorsqu'elle est dispersée dans une solution aqueuse d'antigène contenant un tampon physiologique, s'inverse et se disperse pour donner une dispersion fluide et translucide de microgels de polymères dans l'eau. Par exemple, des gouttes de microgels de polyacrylate de sodium peuvent avoir une taille moyenne comprise entre 0,5 et 5 micromètres, par exemple environ 2 micromètres.

L'invention a pour objet, un procédé de préparation d'un vaccin mettant en œuvre un latex inverse. Plus particulièrement, l'invention a pour objet un procédé de préparation d'un vaccin, comprenant :
- une étape a) de préparation d'une composition adjuvante par dispersion dans une solution aqueuse physiologiquement acceptable, d'au moins un latex inverse ou d'une poudre de polymère résultant de l'atomisation dudit latex inverse ;
- une étape b) de mélange de la composition obtenue à l'étape a) dans un milieu antigénique, destiné à former une composition vaccinale.

Par latex inverse on entend une émulsion eau-dans-huile de polymère, dans laquelle la phase aqueuse, dispersée dans la phase huileuse continue, contient ledit polymère. Le latex inverse, résultant de la polymérisation en émulsion inverse se présente donc sous la forme d'une émulsion liquide. Une atomisation ultérieure de ce latex inverse peut, si nécessaire ou si désirée, être effectuée pour former ladite poudre de polymère. L'atomisation est par exemple décrite dans la demande de brevet européen EP 1 496 081.

Par solution aqueuse physiologiquement acceptable, on désigne dans le cadre de la présente invention les solutions aqueuses qui sont aptes à être utilisées dans la préparation de vaccins, qu'il s'agisse par exemple d'eau de qualité conforme aux pharmacopées notamment européenne ou américaine comme par exemple les sérums physiologiques, ou de solutions salines et/ou le cas échéant hydro-alcooliques conformes aux dites pharmacopées.

Par composition adjuvante on désigne une composition adjuvante d'immunité.

Au moins un tensioactif de caractère hydrophile peut également être ajouté préalablement à cette étape a) de façon à améliorer la stabilité d'une telle dispersion dans le temps.

Par tensioactif de caractère hydrophile, on désigne des agents tensioactifs émulsifiants possédant une valeur de HLB suffisamment élevée, comprise entre 10 et 15, pour fournir des émulsions huile dans l'eau stables, tels que les esters de manithan éthoxylés, les esters de sorbitan éthoxylés comme, par exemple, l'oléate de sorbitan éthoxylé avec 20 moles d'oxyde d'éthylène, commercialisé par la société SEPPIC sous le nom de MONTANOX^{™}80.

Par milieu antigénique on désigne un milieu comprenant au moins un antigène ou au moins un générateur in vivo d'un composé comprenant une séquence d'acides aminés. Par antigène ou au moins un générateur in vivo d'un composé comprenant une séquence d'acides aminés, on désigne soit des micro-organismes tués, tels que les virus, les bactéries ou les parasites, soit des fractions purifiées de ces micro-organismes, soit des micro-organismes vivants dont le pouvoir pathogène a été atténué. A titre d'exemples de virus pouvant constituer un antigène selon la présente invention, il y a le virus de la rage, les herpès virus, tels que le virus de la maladie d'Aujeszky, les orthomixovirus tels que Influenzae, les picornavirus tels que le virus de la fièvre aphteuse ou les rétrovirus tels que les VIH. A titre de micro-organisme du type bactérien pouvant constituer un antigène selon la présente invention, on peut citer E. Coli, et ceux des genres Pasteurella, Furonculosis, Vibriosis, Staphylococcus et Streptococcus. A titre d'exemples de parasites, il y a ceux des genres Trypanosoma, Plasmodium et Leishmania. On peut aussi citer les virus recombinants notamment les virus non enveloppés, tels que les adénovirus, le virus de la vaccine, le virus Canarypox, les herpès virus ou les baculovirus. On désigne aussi un vecteur recombinant viral non enveloppé vivant, dont le génome contient, insérée de préférence dans une partie non essentielle pour la réplication du virus enveloppé correspondant, une séquence codant pour une sous - unité antigénique induisant une synthèse d'anticorps et/ou un effet protecteur contre le susdit virus enveloppé ou micro-organisme pathogène ; ces sous - unités antigéniques peuvent être par exemple, une protéine, une glycoprotéine, un peptide ou une fraction peptidique et/ou protectrice contre une infection par un micro-organisme vivant tel un virus enveloppé, une bactérie ou un parasite. Le gène exogène inséré dans le micro-organisme peut être, par exemple, issu d'un virus Aujeszky ou HIV.

On peut citer notamment un plasmide recombinant constitué d'une séquence de nucléotides, dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte.

Par générateur "in vivo" d'un composé comprenant une séquence d'acides aminés, on désigne tout un produit biologique capable d'exprimer ledit composé dans l'organisme hôte dans lequel on a introduit ledit générateur in vivo. Le composé comprenant la séquence d'acides aminés, peut être une protéine, un peptide ou une glycoprotéine. Ces générateurs in vivo sont généralement obtenus par des procédés issus du génie génétique. Plus particulièrement, ils peuvent consister en des micro-organismes vivants, généralement un virus, jouant le rôle de vecteur recombinant, dans lequel est insérée une séquence nucléotidique, notamment un gène exogène. Ces composés sont connus en tant que tels et utilisés notamment comme vaccin sous unitaire recombinant. A cet égard, on peut se référer à l'article de M. ELOIT et al, Journal of virology (1990) 71, 2925-2431 et aux demandes internationales de brevet publiées sous les numéros WO-A-91/00107 et WO-A-94/16681. Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par LIN et al, Circulation 82:2217,2221 ; COX et al, J. of VIROL, Sept. 1993, 67, 9, 5664-5667 et dans la demande internationale publiée sous le numéro WO 95/25542. Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune,
(ii) avoir une action curative vis-à-vis d'une maladie, essentiellement une maladie d'ordre fonctionnel, qui s'est déclenchée chez l'organisme hôte. Dans ce cas, le générateur in vivo permet un traitement de l'hôte, du type thérapie génique.

A titre d'exemple, une telle action curative peut consister en une synthèse par le générateur in vivo de cytokines, comme les interleukines, notamment l'interleukine 2. Celles-ci permettent le déclenchement ou le renforcement d'une réaction immune visant à l'élimination sélective des cellules cancéreuses.

La composition vaccinale telle que définie ci-dessus, comprend une concentration en antigène qui dépend de la nature de cet antigène et de la nature du sujet traité. Il est toutefois particulièrement remarquable qu'un adjuvant selon l'invention, permette de diminuer notablement la dose habituelle d'antigène requise. La concentration adéquate d'antigène peut être déterminée de manière classique par l'homme du métier. Généralement, cette dose est de l'ordre de 0,1 µg / cm³ à 1 g / cm³ plus généralement comprise entre 1 µg / cm³ et 100 mg / cm³. La concentration dudit générateur in vivo dans la composition selon l'invention dépend, là encore, notamment de la nature dudit générateur et de l'hôte dans lequel il est administré. Cette concentration peut être aisément déterminée par l'homme du métier, sur la base d'expérience de routine. A titre indicatif, lorsque le générateur in vivo est un micro-organisme recombinant, sa concentration dans la composition selon l'invention en général comprise entre 10² et 10¹⁵ microorganismes / cm³ et de préférence entre 10⁵ et 10¹² microorganismes / cm³. Lorsque le générateur in vivo est un plasmide recombinant, sa concentration dans la composition obtenue selon le procédé objet de l'invention peut être comprise entre 0,01 g / dm³ et 100 g / dm³. Le vaccin, tel que défini précédemment, est préparé en mélangeant la phase adjuvante et la phase antigénique, en ajoutant éventuellement de l'eau ou un milieu diluant pharmaceutiquement acceptable.

La phase continue huileuse, utilisée pour préparer le latex inverse mis en œuvre au cours de l'étape a) du procédé objet de la présente invention, comprend un ou plusieurs composés choisis parmi les huiles d'origine minérale, végétale ou animale, les esters alkyliques desdites huiles, les esters alkyliques d'acides gras ou les éthers alkyliques d'acides gras, les esters d'acides gras et de polyols ou les éthers d'alcools gras et de polyols, les huiles de synthèse.

Une huile minérale commerciale peut également être utilisée, c'est à dire une huile minérale commerciale contenant des hydrocarbures saturés comme les paraffines, les isoparaffines, les cycloparaffines, présentant à température ambiante, une densité entre 0.7 et 0.9 et un point d'ébullition supérieur à 180°C, telles que par exemple l'EXXSOL^{™} D 100 S, ou le MARCOL^{™}52 commercialisées par la société EXXON CHEMICAL, l'isohexadécane ou l'isododécane, soit un mélange de plusieurs de ces huiles.

Selon un aspect préféré de la présente invention, la phase huile est constituée de MARCOL^{™} 52 ou d'isohexadécane ; l'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER. Le MARCOL^{™} 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Comme exemples d'huiles d'origine végétale, il y a l'huile d'arachide, l'huile d'olive, l'huile de sésame, l'huile de soja, l'huile de germes de blé, l'huile de pépins de raisin, l'huile de tournesol, l'huile de ricin, l'huile de lin, l'huile de soja, l'huile de maïs, l'huile de coprah, l'huile de palme, l'huile de noix, l'huile de noisette, l'huile de colza ou encore le squalène ou le squalane d'origine végétale commercialisé en France par la société SOPHIM, sous le nom PHYTOSQUALAN^{™}, identifié dans Chemical Abstracts par le numéro RN = 111-01-3, et qui consiste en un mélange d'hydrocarbures contenant plus de 80% en poids de 2,6,10,15,19,23 - hexaméthyl tétracosane .

Comme exemples d'huiles d'origine animale, il y a l'huile de spermaceti, l'huile de suif, le squalane ou le squalène extraits des foies de poissons.

Comme exemples d'esters alkyliques d'huiles, il y a les esters méthyliques éthyliques, propyliques linéaires ou ramifiés ou butyliques, linéaires ou ramifiés, desdites huiles.

Comme acides gras appropriés à la préparation des esters cités ci-dessus, il y a plus particulièrement, ceux comportant de 12 à 22 atomes de carbone, tels que par exemple, l'acide myristique, l'acide palmitique, l'acide oléique, l'acide ricinoléique ou l'acide isostéarique et avantageusement un acide gras liquide à 20°C.

Comme exemples d'esters d'acides gras ou d'éthers d'acides gras, il y a les esters alkyliques d'acides gras, tels que, l'oléate d'éthyle, l'oléate de méthyle, le myristate d'isopropyle ou le palmitate d'octyle, les esters d'acides gras et de polyols ou les éthers d'alcools gras et de polyols, tels que les monoglycérides d'acides gras, les diglycérides d'acides gras, les triglycérides d'acides gras, les esters d'acides gras avec un polyglycérol ou les esters d'acides gras et de propylèneglycol, et plus particulièrement les esters d'acides gras avec un hexol, tel que par exemple le sorbitol ou le mannitol, les esters d'acides gras avec un anhydride d'hexol, comme le sorbitane ou le mannitane.

Comme exemples d'huile de synthèse, il y a le polydécène hydrogéné ou le polyisobutène hydrogéné, commercialisé en France par la société Ets B. Rossow et Cie sous le nom PARLEAM - POLYSYNLANE^{™}, qui est cité par Michel et Irene Ash, « Thesaurus of Chemical Products », Chemical Publishing Co, Inc. 1986 Volume I, page 211 (ISBN 0 7131 3603 0).

Dans le cadre de la présente invention, la phase continue huileuse, utilisée pour préparer le latex inverse mis en œuvre au cours de l'étape a) du procédé objet de la présente invention, peut comprendre un seul des composés cités ci-dessus ou bien un mélange de plusieurs des composés cités ci-dessus.

La proportion de la phase huileuse dans le latex inverse est comprise entre 10% et 50% massique, et de préférence entre 15% et 25% massique, de la masse totale du latex inverse.

Ledit au moins un polymère obtenu par polymérisation en émulsion inverse conduisant au latex inverse à la base de la préparation de la composition adjuvante préparée lors de l'étape a) du procédé objet de l'invention est choisi parmi les polyélectrolytes anioniques, branchés ou réticulés, choisis parmi les copolymères de l'acide acrylique et de l'acide-2-méthyl-[(1-oxo-2-propényl) amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique partiellement ou totalement salifié, l'homopolymère de l'acide acrylique partiellement ou totalement salifié, l'homopolymère de l'acide méthacrylique partiellement ou totalement salifié, les copolymères du chlorure d'acryloyl éthyl triméthyl ammonium et de l'acrylamide, les copolymères de l'AMPS et de la vinylpyrolidone, les copolymères de l'AMPS et de la N-m éthyl acrylamide, les copolymères de l'AMPS et du N,N-diméthyl acrylamide, les copolymères de l'AMPS et du methacrylamide, les copolymères de l'AMPS et du N-isopropylacrylamide, les copolymères de l'AMPS et du N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], les copolymères de l'acide acrylique et de l'acrylamide, les copolymères de l'acide acrylique et de la N-méthyl acrylamide, les copolymères de l'acide acrylique et du N,N-diméthyl acrylamide, les copolymères de l'acide acrylique et du methacrylamide, les copolymères de l'acide acrylique et du N-isopropylacrylamide, les copolymères de l'acide acrylique et du N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités, à bas gradient de vitesse, très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

Lorsque ce polyélectrolyte est réticulé, il l'est plus particulièrement avec un composé diéthylènique ou polyéthylènique dans la proportion molaire exprimée par rapport aux monomères mis en oeuvre, de 0,005% à 1%, et de préférence de 0,01% à 0,2% et, plus particulièrement de 0,01% à 0,1%. De préférence l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate ou le méthylène-bis-(acrylamide) , allylsucrose.

Lorsque le latex inverse est mis en œuvre lors de l'étape de préparation de la composition adjuvante préparée dans l'étape a) objet du procédé de l'invention, il comprend généralement de 1% à 5% massique d'un système émulsionnant de type eau dans huile (E/H).

Par système émulsionnant de type eau dans huile (E/H), on désigne dans la définition précédente, soit un seul tensioactif soit un mélange de tensioactifs à condition que ledit mélange ait une valeur de HLB suffisamment faible pour induire des émulsions eau dans huile. Comme agent émulsionnant de type eau - dans huile, il y a par exemple les esters de sorbitan, comme l'oléate de sorbitan, comme celui commercialisé par la société SEPPIC sous le nom MONTANE^{™} 80, l'isostéarate de sorbitan, comme celui commercialisé par la société SEPPIC sous le nom MONTANE^{™} 70 ou le sesquioléate de sorbitan comme celui commercialisé par la société SEPPIC sous le nom MONTANE^{™} 83. Il y a aussi certains esters de sorbitan polyéthoxylés, par exemple le monooléate de sorbitan pentaéthoxylé comme celui commercialisé par la société SEPPIC sous le nom MONTANOX^{™} 81 ou l'isostéarate de sorbitan pentaéthoxylé comme celui commercialisé sous le nom MONTANOX^{™} 71 par la société SEPPIC. Il y a encore l'alcool oléocétylique diéthoxylé comme celui commercialisé sous le nom SlMULSOL^{™} OC 72 par la société SEPPIC,les polyesters de poids moléculaire compris entre 1000 et 3000, produits de la condensation entre un acide poly(isobutènyl) succinique ou son anhydride et la diethanol amine tels que l'HYPERMER^{™} 2296 commercialisé par la société UNIQEMA ou enfin les copolymères à blocs de poids moléculaire compris entre 2500 et 3500, comme l'HYPERMER^{™} B246 commercialisé par la société UNIQEMA ou la SlMALlNE^{™} IE 200 commercialisé par la société SEPPIC.

Lorsque le latex inverse est mis en œuvre lors de l'étape de préparation de la composition adjuvante préparée dans l'étape a) objet du procédé de l'invention, il comprend généralement de 2% à 8% massique d'un système émulsionnant de type huile dans eau (H/E).

Par système émulsionnant de type huile dans eau (H/E), on désigne dans la définition précédente, soit un seul tensioactif soit un mélange de tensioactifs à condition que ledit mélange ait une valeur de HLB suffisamment élevée pour induire des émulsions huile dans eau. Comme agent émulsionnant de type huile dans eau, il y a par exemple les esters de sorbitan éthoxylés comme l'oléate de sorbitan polyéthoxylé avec 20 moles d'oxyde d'éthylène, commercialisé par la société SEPPIC sous le nom de MONTANOX^{™} 80, le laurate de sorbitan polyéthoxylé avec 20 moles d'oxyde d'éthylène , commercialisé par la société SEPPIC sous le nom de MONT ANOX^{TM} 20, l'huile de ricin polyéthoxylé avec 40 moles d'oxyde d'éthylène commercialisé sous le nom SIMULSOL^{™} OL50, l'alcool oléodécylique décaéthoxylé, commercialisé par la société SEPPIC sous le nom SIMULSOL^{™} OC 710, l'alcool laurique heptaéthoxylé commercialisé sous le nom SIMULSOL^{™} P7 ou le monostéarate de sorbitan polyéthoxylé avec 20 moles d'oxyde d'éthylène commercialisé par la société SEPPIC sous le nom MONTANOX^{™} 60.

Un polymère obtenu par polymérisation en émulsion, conduisant au latex inverse à la base de la préparation de la composition adjuvante préparée lors de l'étape a) du procédé objet de l'invention, avantageusement utilisé est choisi parmi le groupe constitué de l'homopolymère de l'acide acrylique totalement ou partiellement salifié, l'homopolymère de l'acide méthacrylique partiellement ou totalement salifié, les copolymères de l'acide acrylique et de l'acide-2-méthyl-[(1-oxo-2-propényl)amino]1-propane sulfonique, les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), et encore plus avantageusement le polyacrylate de sodium.

La proportion massique dudit au moins un latex inverse dans la composition adjuvante préparée lors de l'étape a) du procédé objet de l'invention, est comprise entre 0,5% et 30% de la masse totale de la composition adjuvante, et préférentiellement entre 5% et 15% de la masse totale de la composition adjuvante.

Lors de l'étape a) du procédé objet de la présente invention, une substance immunostimulante peut être ajoutée. La substance immunostimulante est par exemple choisie parmi un ou plusieurs agents stimulants immunitaires conventionnels, tel l'Avridine^{™}, la N,N-dioctadecyl-N',N'-bis(2-hydroxyéthyl) propanediamine, les dérivés du MDP (muramyl dipeptide), notamment le thréonyl-MDP, les dérivés de l'acide mycolique ou les dérivés du Lipide A. Une telle substance est plus particulièrement une substance ionisée. La substance ionisée est par exemple choisie parmi un ou plusieurs sels organiques de cations métalliques hydrosolubles, tel que par exemple le gluconate de calcium, le gluconate de manganèse, le salicylate d'aluminium ou l'acétate d'aluminium soluble. Lorsque la composition adjuvante selon l'invention comprend, un sel pharmaceutiquement acceptable, celui-ci est à une concentration de 0,02 à 3000 mg/ cm³, de préférence 0,1 à 1000 mg/cm³, plus préférentiellement de 0,1 à 150 mg/cm³. Des sels insolubles généralement utilisés comme adjuvants d'immunité, tels que l'hydroxyde d'aluminium ou le phosphate de calcium peuvent également être utilisés.

Selon un aspect de l'invention, lors de l'étape a), au moins un tensioactif à caractère hydrophile destiné à stabiliser l'émulsion est ajouté. Le HLB dudit au moins un tensioactif à caractère hydrophile est compris entre 10 et 15.

La composition préparée à l'étape a) comporte entre 0,1% et 15% dudit au moins un tensioactif et de préférence entre 0,1% et 5% dudit au moins un tensioactif.

Au sens de la présente invention, le nombre HLB d'un tensioactif est calculé par la formule HLB = 20 (1-Iₛ/Iₐ), dans laquelle Iₛ représente l'indice de saponification et Iₐ, l'indice d'acide de l'acide gras utilisé pour la préparation dudit tensioactif. Dans le cas d'un mélange de tensioactifs, le HLB du mélange est la somme pondérée des HLB de chaque tensioactif. Ces deux indices, de saponification et d'acide, sont déterminés par des méthodes décrites dans la Pharmacopée européenne.

Selon un autre aspect particulier de l'invention, la composition obtenue lors de l'étape a) du procédé objet de la présente invention, présente une teneur massique en phase huileuse comprise entre 0,1% et 5% de la masse totale de la composition obtenue lors de l'étape a) du procédé objet de la présente invention, et de préférence entre 0,2% et 1% massique.

Les agents tensioactifs à caractère hydrophile mis en œuvre sont généralement choisis parmi les corps gras modifiés.

Les corps gras modifiés utilisés dans le cadre de la présente invention, peuvent être d'origine minérale végétale ou animale. Comme corps gras modifiés d'origine minérale, il y a les huiles d'origine pétrolière.

Comme corps gras modifiés d'origine végétale, il y a les huiles végétales modifiées, par exemple, les huiles modifiées d'arachide, d'olive, de sésame, de soja, de germes de blé, de pépins de raisin de tournesol, de ricin, de lin, de soja, de maïs, de coprah, de palme, de noix, de noisettes ou de colza.

Comme corps gras modifiés d'origine animale, il y a par exemple le squalane modifié, le squalène modifié, l'huile modifiée de spermaceti ou l'huile modifiée de suif.

Par corps gras modifiés, on désigne notamment les dérivés alcoxylés de corps gras et plus particulièrement les dérivés alcoxylés d'huiles ou les dérivés alcoxylés d'esters alkyliques d'huiles et plus particulièrement, les dérivés éthoxylés et/ou propoxylés d'huiles ou les dérivés éthoxylés et/ou propoxylés des esters méthyliques, éthyliques, propyliques linéaires ou ramifiés ou butyliques, linéaires ou ramifiés, desdites huiles. L'invention a plus spécifiquement pour objet une composition telle que définie précédemment, dans laquelle le corps gras modifié est choisi parmi les dérivés éthoxylés d'huiles ayant un nombre de moles d'oxyde d'éthylène compris entre 1 et 10.

Par corps gras modifiés on désigne aussi, les esters d'acides gras et de polyols ou les éthers d'alcools gras et de polyols, et plus particulièrement, les esters d'acides gras avec un hexol, tel que par exemple le sorbitol ou le mannitol ou les esters d'acides gras avec un anhydride d'hexol, comme le sorbitane ou le mannitane, les dérivés alcoxylés d'esters d'acides gras et de polyols ou les dérivés alcoxylés d'éthers d'alcools gras et de polyols, comme les triglycérides d'acides gras alcoxylés, les esters alcoxylés de polyglycérol d'acides gras, et plus particulièrement les esters alcoxylés d'acides gras avec un hexol, tel que par exemple le sorbitol ou le mannitol ou les esters alcoxylés d'acides gras avec un anhydride d'hexol, comme le sorbitane ou le mannitane ayant un nombre de moles d'oxyde d'éthylène compris entre 1 et 20.

Par esters d'acides gras et de polyols, on désigne dans le cadre de la présente invention, les monoesters d'acides gras et de polyols ou les polyesters d'acides gras et de polyol tels que par exemple les diesters d'acides gras et de polyols ou les triesters d'acides gras et de polyols. Il en est de même pour les dérivés polyalcoxylés desdits esters.

Par éthers d'acides gras et de polyols, on désigne dans le cadre de la présente invention, les monoéthers d'acides gras et de polyols ou les polyéthers d'acides gras et de polyol tels que par exemple les diéthers d'acides gras et de polyols ou les triéthers d'acides gras et de polyols. Il en est de même pour les dérivés polyalcoxylés desdits éthers.

Les corps gras modifiés peuvent être choisis parmi les dérivés éthoxylés d'esters d'acides gras et de polyols ou les dérivés éthoxylés d'éthers d'alcools gras et de polyols, et plus particulièrement, les esters éthoxylés d'acides gras avec un hexol, tel que par exemple le sorbitol ou le mannitol ou les esters éthoxylés d'acides gras avec un anhydride d'hexol, comme le sorbitane ou le mannitane ayant un nombre de moles d'oxyde d'éthylène compris entre 5 et 10.

Comme acides gras appropriés à la préparation des corps gras modifiés décrits ci-dessus, il y a ceux comportant en moyenne de 12 à 22 atomes de carbone, tels que par exemple, ceux comportant de 16 à 18 atomes de carbone, comme l'acide oléique, l'acide ricinoléique ou l'acide isostéarique et avantageusement les acides gras liquides à 20 °C.

Selon un aspect de l'invention, lors de l'étape b), au moins un immunostimulant choisi parmi les saponines, les huiles animales et/ou végétales et/ou minérales et/ou de synthèse, les tensioactifs, l'hydroxyde d'aluminium, les lécithines et les dérivés de lécithine est ajouté.

Selon un aspect particulier de l'invention, le vaccin obtenu lors de l'étape b), comprend entre 10% et 20% de la préparation obtenue à l'étape a) et entre 80% et 90% d'un milieu antigénique.

L'invention a également pour objet une composition vaccinale comprenant entre 10% et 20% d'une préparation obtenue selon l'étape a) du procédé tel que décrit précédemment et entre 80% et 90% d'un milieu antigénique.

La composition vaccinale telle que décrite ci-dessus peut être utilisée comme médicament préventif ou curatif. Selon la nature de l'antigène ou du générateur in vivo, une composition selon l'invention peut être administrée à des poissons, des crustacés tels que les crevettes, des volailles, notamment, des oies, des dindes, des pigeons et des poulets, aux canidés tels le chien, aux félidés tels le chat, aux porcs, aux primates, aux bovidés, aux ovidés, aux chevaux, à des rongeurs tels que le rat, des lagomorphes tels que le lapin, des caprins tels que les chèvres, des gros mammifères tels que les éléphants. La composition selon l'invention peut être également administrée à l'homme.

L'administration de la composition peut se faire de manière classique par voie parentérale, notamment par injection sous-cutanée, intramusculaire ou intrapéritonéale. Elle peut aussi se faire par voie orale, nasale, oculaire, par immersion ou par balnéation.

L'invention a enfin pour objet l'utilisation d'une composition adjuvante obtenue selon l'étape a) du procédé tel que décrit précédemment pour la préparation d'une composition vaccinale.

Les pourcentages utilisés tout au long de la demande représentent des proportions massiques.

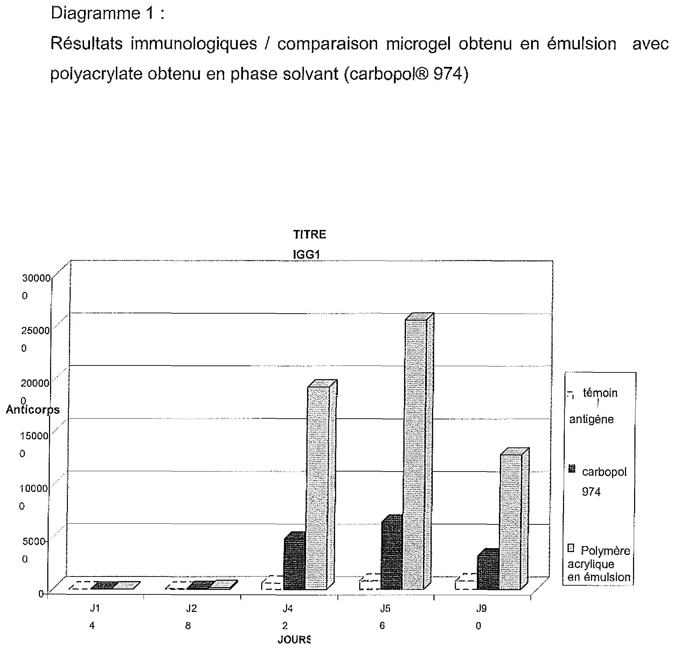

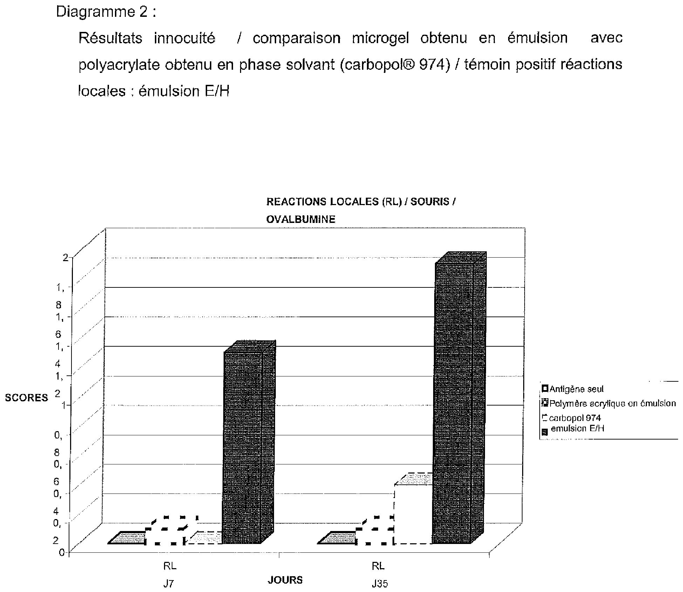

De manière fort surprenante, la comparaison des propriétés adjuvantes de dispersions de latex inverses par rapport à des polymères de même composition chimique mais obtenus par synthèse en phase solvant suivie d'une précipitation, démontre des effets adjuvants très supérieurs tant sur la réponse humorale (diagramme 1) que cellulaire accompagnée d'une grande innocuité (diagramme 2).

Les exemples suivants illustrent l'invention sans toutefois la limiter.

### Exemple 1 : Composition adjuvante à base de polyacrylate de sodium obtenu en émulsion eau dans huile minérale.

On utilise une huile minérale fluide « Marcol^{®} 52 » fournie par EXXON. L'émulsion de polymère obtenue après polymérisation se présente sous la forme d'un gel huileux blanc et visqueux. Sa composition est de 30% d'huile minérale (la phase continue), 5% de tensioactif destinés à stabiliser l'émulsion et 25% de polyacrylate de sodium et 40% d'eau. Au contact d'une solution antigénique aqueuse tamponnée à pH 7, à 3%, l'émulsion polymère dans huile s'inverse pour donner une dispersion fluide de polymère contenant des traces d'huile.

### Exemple 2 : Adjuvant pour vaccin à base de polyacrylate de sodium obtenu par polymérisation en émulsion.

Une dispersion dans l'eau contenant 10% du polymère décrit dans l'exemple 1 est préparée avec une agitation mécanique suffisante. Le réglage de la viscosité peut être réalisé par l'ajout de substances ionisées (sels) inertes (exemple chlorure de sodium) ou immunostimmlante (exemple gluconate de manganèse). Ce gel, prêt à l'emploi, peut être redispersé à la concentration nécessaire dans le milieu antigénique considéré. L'ajout, dans le gel, de tensioactifs supplémentaires destinés à stabiliser la dispersion de l'huile dans l'eau peut aussi être effectué.

### Exemple 3 : Vaccin contenant du polyacrylate de sodium obtenu par polymérisation en émulsion.

Un vaccin est constitué de 85% de milieu antigénique dans lequel on ajoute 15% de la préparation décrite à l'exemple 2. L'ajout d'autres immunostimulants comme par exemple les saponines, huiles animales et/ou végétales et/ou minérales et/ou de synthèse, tensioactifs, hydroxyde d'aluminium, lécithines et dérivés de lécithine, est possible.

## Revendications

1. Procédé de préparation d'un vaccin, comprenant :
- Une étape a) de dispersion dans une solution aqueuse physiologiquement acceptable, d'-un latex inverse d'un homopolymère de l'acide acrylique partiellement ou totalement salifié sous forme de sel de sodium, branché ou réticulé, ainsi que d'un tensioactif à caractère hydrophile, dont le HLB est compris entre 10 et 15, pour obtenir une composition adjuvante comprenant pour 100% de sa masse, entre 0,5% et 30% massique dudit latex inverse et entre 0,1% et 15% dudit tensioactif hydrophile ;
- Une étape b) de mélange de la composition adjuvante obtenue à l'étape a) dans un milieu antigénique, pour former ledit vaccin.

2. Procédé selon la revendication 1, dans lequel la composition adjuvante obtenue à l'étape a) comprend pour 100% de sa masse, entre 5% et 15% massique dudit latex inverse et entre 0,1% et 5% dudit tensioactif hydrophile.

3. Procédé selon l'une quelconque des revendications 1 ou 2, dans lequel la composition adjuvante obtenue à l'étape a) présente une teneur massique en phase huileuse comprise entre 0,1% et 5% massique.

4. Procédé selon la revendication 3, dans lequel la composition adjuvante obtenue à l'étape a) présente une teneur massique en phase huileuse comprise entre 0,2% et 1% massique.

5. Procédé selon l'une quelconque des revendications 1 à 4, dans lequel au moins une substance immunostimulante est ajoutée lors de l'étape a).

6. Procédé selon l'une quelconque des revendications 1 à 5, dans lequel l'étape b) consiste à mélanger de 10% à 20% de la composition adjuvante obtenue à l'étape a) avec 80% à 90% d'un milieu antigénique.

7. Procédé selon l'une quelconque des revendications 1 à 6, dans lequel au moins un immunostimulant choisi parmi les saponines, les huiles animales et/ou végétales et/ou minérales et/ou de synthèse, les tensioactifs, l'hydroxyde d'aluminium, les lécithines et les dérivés de lécithine est ajouté lors de l'étape b).

## Patentansprüche

1. Ein Verfahren zur Herstellung eines Impfstoffes, umfassend:
- Einen Schritt a) des Dispergierens eines Inverse-Latex eines teil- oder vollsalifizierten Acrylsäurehomopolymers in Form eines Natriumsalzes, verzweigt oder vernetzt, sowie eines hydrophilen Tensids, dessen HLB-Wert zwischen 10 und 15 liegt, in einer physiologisch akzeptablen wässrigen Lösung, um eine Adjuvanszusammensetzung zu erhalten, die bezogen auf 100 Gew.-% ihrer Masse zwischen 0,5 Masseprozent und 30 Masseprozent des besagten Inverse-Latex und zwischen 0,1 Masseprozent und 15 Masseprozent des besagten hydrophilen Tensids umfasst;
- Einen Schritt b) des Mischens der in Schritt a) erhaltenen Adjuvanszusammensetzung in einem antigenen Medium, um den besagten Impfstoff zu bilden.

2. Verfahren nach Anspruch 1, wobei die in Schritt a) erhaltene Adjuvanszusammensetzung bezogen auf 100 Gew.-% ihrer Masse zwischen 5 Masseprozent und 15 Masseprozent des besagten Inverse-Latex und zwischen 0,1 Masseprozent und 5 Masseprozent des besagten hydrophilen Tensids umfasst.

3. Verfahren nach einem der Ansprüche 1 oder 2, wobei die in Schritt a) erhaltene Adjuvanszusammensetzung einen Massemengenanteil an Ölphase zwischen 0,1 Masseprozent und 5 Masseprozent aufweist.

4. Verfahren nach Anspruch 3, wobei die in Schritt a) erhaltene Adjuvanszusammensetzung einen Massemengenanteil an Ölphase zwischen 0,2 Masseprozent und 1 Masseprozent aufweist.

5. Verfahren nach einem der Ansprüche 1 bis 4, wobei mindestens eine immunstimulierende Substanz während Schritt a) zugegeben wird.

6. Verfahren nach einem der Ansprüche 1 bis 5, wobei Schritt b) das Mischen von 10% bis 20% der in Schritt a) erhaltenen Adjuvanszusammensetzung mit 80% bis 90% eines antigenen Mediums umfasst.

7. Verfahren nach einem der Ansprüche 1 bis 6, wobei mindestens eine immunstimulierende Substanz, ausgewählt aus Saponinen, tierischen und/oder pflanzlichen und/oder mineralischen und/oder synthetischen Ölen, Tensiden, Aluminiumhydroxid, Lecithinen und Lecithinderivaten, während Schritt b) zugegeben wird.

## Claims

1. A process for preparing a vaccine, comprising:
- A step a) of dispersing, in a physiologically acceptable aqueous solution, an inverse latex of a partially or totally salified acrylic acid homopolymer in the form of a sodium salt, branched or cross-linked, as well as a hydrophilic surfactant, the HLB of which is between 10 and 15, to obtain an adjuvant composition comprising, for 100% of its weight, between 0.5% and 30% by weight of said inverse latex and between 0.1% and 15% of said hydrophilic surfactant;
- A step b) of mixing the adjuvant composition obtained in step a) into an antigenic medium, to form said vaccine.

2. The process according to claim 1, wherein the adjuvant composition obtained in step a) comprises, for 100% of its weight, between 5% and 15% by weight of said inverse latex and between 0.1% and 5% of said hydrophilic surfactant.

3. The process according to any one of claims 1 or 2, wherein the adjuvant composition obtained in step a) exhibits an oil phase content by weight comprised between 0.1% and 5% by weight.

4. The process according to claim 3, wherein the adjuvant composition obtained in step a) exhibits an oil phase content by weight comprised between 0.2% and 1% by weight.

5. The process according to any one of claims 1 to 4, wherein at least one immunostimulating substance is added during step a).

6. The process according to any one of claims 1 to 5, wherein step b) consists of mixing 10% to 20% of the adjuvant composition obtained in step a) with 80% to 90% of an antigenic medium.

7. The process according to any one of claims 1 to 6, wherein at least one immunostimulating substance chosen from saponins, animal and/or vegetable and/or mineral and/or synthetic oils, surfactants, aluminum hydroxide, lecithins and lecithin derivatives is added during step b).
